Europäisches Patentamt

European Patent Office

Office européen des brevets

(11) Veröffentlichungsnummer: **0 221 888 B1**

(12) **EUROPÄISCHE PATENTSCHRIFT**

(45) Veröffentlichungstag der Patentschrift: **24.07.91**

(51) Int. Cl.⁵: **C07C 55/07**, C07C 51/41, C07C 51/43

(21) Anmeldenummer: **86890293.3**

(22) Anmeldetag: **30.10.86**

(54) **Verfahren zur Entfernung von Oxalsäure aus sauren Gärflüssigkeiten von citronensäurebildenden Mikroorganismen.**

(30) Priorität: **07.11.85 AT 3212/85**

(43) Veröffentlichungstag der Anmeldung:
**13.05.87 Patentblatt 87/20**

(45) Bekanntmachung des Hinweises auf die Patenterteilung:
**24.07.91 Patentblatt 91/30**

(84) Benannte Vertragsstaaten:
**DE FR GB IT NL SE**

(56) Entgegenhaltungen:
**DE-A- 230 722**

**CHEMICAL ABSTRACTS, Band 76, Nr. 9, 28. Februar 1972, Seite 339, Zusammenfassung Nr. 45678n, Columbus, Ohio, US; S. EIICHI: "Oxalic acid. IV. Decomposition of calcium oxalate with sulfuric acid"**

**CHEMICAL ABSTRACTS, Band 80, Nr. 16, 22. April 1974, Seiten 105,106, Zusammenfassung Nr. 84948c, Columbus, Ohio, US; H.D. WALLENSTEIN et al.: "Acids in beet and juices. 6. Determination of acids which exist as difficulty soluble salts"**

**CHEMICAL ABSTRACTS, Band 102, Nr. 24, 17. Juni 1985, Seite 317, Zusammenfassung Nr. 208821a, Columbus, Ohio, US**

(73) Patentinhaber: **VOGELBUSCH GESELLSCHAFT m.b.H.**
**Blechturmgasse 11**
**A-1050 Wien(AT)**

(72) Erfinder: **Salzbrunn, Wolfgang, Dr.**
**Bräunlichgasse 11**
**A-2700 Wr. Neustadt(AT)**
Erfinder: **Kominek, Jiri, Dipl.-Ing.**
**Zeltgasse 2/1/4**
**A-1080 Wien(AT)**
Erfinder: **Röhr, Max, Prof.**
**Asenbauergasse 32-34**
**A-1230 Wien(AT)**

(74) Vertreter: **Wolfram, Gustav, Dipl.-Ing.**
**Schwindgasse 7 P.O. Box 205**
**A-1041 Wien(AT)**

## Beschreibung

Die Erfindung betrifft ein Verfahren zur Entfernung von Oxalsäure aus sauren Gärflüssigkeiten von citronensäurebildenden Mikroorganismen durch Fällung der Oxalsäure als schwerlösliches Calciumoxalat und Abtrennung des Calciumoxalates.

Citronensäurehältige Gärflüssigkeiten, welche durch Fermentation mittels eines Mikroorganismus, meist des Pilzes Aspergillus niger, nach einem Oberflächen- oder Submersverfahren erhalten werden, sind praktisch immer mit mehr oder weniger großen Mengen an Begleitstoffen verunreinigt, von denen besonders die Oxalsäure unerwünscht ist (L.M. Miall, in Economic Microbiology Vol. 2 (A.H. Rose edit.) p. 68, Acad. Press London-New York-San Francisco 1978; L.B. Lockwood, in Microbial Technology Vol. 1 (H.J. Peppler and D. Perlman edit.) p. 356, Acad. Press., New York-San Francisco-London, 1979). Soll Citronensäure den Anforderungen des jeweiligen Arzneibuches genügen, so darf sie keine oder höchstens Spuren von Oxalsäure enthalten, d.h. letztere darf mit den im Arzneibuch vorgeschriebenen Methoden nicht nachweisbar sein oder ein bestimmter, sehr geringer Gehalt darf nicht überschritten werden (vgl. G. Schulz, J. Rauch, in Ullmanns Encyklopädie der technischen Chemie, 4. Aufl., Bd. 9, Seiten 633 und 634, Punkt 5., Verlag Chemie, Weinheim, 1975; R. Schmitz, Chemie-Technik 6 (1977) 255).

Versuche, die besonders bei Hochleistungsstämmen bzw. unter optimalen Produktionsbedingungen (pH-Wert des Gärmediums zwischen 2,0 und 2,5) in störendem Ausmaß auftretende Oxalsäurebildung durch genetische oder produktionstechnische Maßnahmen zu unterdrücken, haben bislang noch nicht zu befriedigenden Ergebnissen geführt, weswegen man darauf angewiesen ist, die gebildete Oxalsäure möglichst am Beginn der Verfahren zur Gewinnung von Citronensäure aus den Gärflüssigkeiten zu entfernen.

Diese Entfernung der Oxalsäure erfolgte bisher meistens nach Abtrennung der Mikroorganismenzellen (z.B. des Pilzmycels) durch Fällung in Form des schwerlöslichen Calciumoxalates mittels Kalkmilch oder Calciumcarbonat (Ullmanns Encyklopädie der technischen Chemie, 3. Auflage, 5. Band, Seite 607, Abb. 3, Urban & Schwarzenberg, 1954). Die dabei gelöst bleibenden geringen Reste an Oxalat werden bei der auf die anschließende Fällung der Citronensäure als Tricalciumcitrat folgenden Zersetzung des Citrates durch hochprozentige Schwefelsäure zerstört, so daß die gewonnene Citronensäure im Sinne der Vorschriften oxalsäurefrei anfällt.

Die beschriebene Vorgangsweise hat jedoch erhebliche Nachteile: Die Fällung erfolgt bei pH-Werten um 3; bei Überschreiten dieses Wertes kommt es bereits zur Mitfällung von Calciumcitrat, das somit für die Citronensäuregewinnung verloren ist. Da Kalkmilch eine starke Base ist, kann es unter den Bedingungen der betrieblichen Praxis sehr leicht zu Citronensäureverlusten durch Überschreiten dieses pH-Wertes kommen.

Die Erfindung bezweckt die Vermeidung dieser Nachteile und stellt sich die Aufgabe, die Oxalsäure möglichst vollständig und betriebssicher aus den Gärflüssigkeiten abzutrennen, dabei aber eine Mitfällung von schwerlöslichen Salzen der Citronensäure ohne aufwendige Überwachungsmaßnahmen sicher zu verhindern und darüber hinaus die Ausbeute an aus den Gärflüssigkeiten gewinnbarer Citronensäure zu erhöhen.

Aus Vorversuchen wurde die Erkenntnis gewonnen, daß für eine praktisch quantitative Fällung des Calciumoxalates nicht der pH-Wert der Gärflüssigkeit maßgebend ist, sondern die erzielte Calciumionenkonzentration bzw. -aktivität. Die erforderliche Calciumionenkonzentration liegt bei Verwendung von Kalkmilch als Fällungsmittel in Citronensäurelösungen erst bei dem weiter oben als kritisch bezeichneten pH-Wert von etwa 3 vor.

Es wurde nun nach einer auch wirtschaftlich vertretbaren Möglichkeit gesucht, die Konzentration an Calciumionen zu erhöhen, dabei aber den pH-Wert der Gärflüssigkeit nicht anzuheben.

Die gestellte Aufgabe wird bei einem Verfahren der eingangs definierten Art erfindungsgemäß dadurch gelöst, daß die Fällung durch Zusatz von Calciumsulfat zu der Gärflüssigkeit vor oder nach Abscheidung der Mikroorganismenzellen vorgenommen wird.

Die Reaktion mit Calciumsulfat entspricht einer doppelten Umsetzung, wie sie in der nachstehenden Gleichung veranschaulicht ist:

$$(COOH)_2 + Ca\,SO_4 \rightleftharpoons Ca\,(COO)_2 + H_2SO_4$$

Wie aus der Gleichung ersichtlich, verschiebt sich durch die freigesetzte Schwefelsäure der pH-Wert günstigerweise weiter in Richtung saurer Reaktion, so daß der pH-Wert-Bereich von unter 3 leicht eingehalten werden kann.

Auch noch bei pH-Werten von unter 1 kann eine ausreichend gute Ausfällung der Oxalsäure erzielt

EP 0 221 888 B1

werden.

Um die Reaktion zu beschleunigen und eine weitestgehende Fällung des Calciumoxalates zu erreichen, ist es von Vorteil, die Fällung bei Temperaturen über 40° C, vorzugsweise zwischen 80 und 90° C, und mit einer Verweilzeit von mindestens 5 min durchzuführen.

Auf diese Weise läßt sich sowohl aus Modellösungen als auch aus Gärflüssigkeiten die Oxalsäure zu über 95 % entfernen.

Vorzugsweise wird Calciumsulfat im stöchiometrischen Überschuß, bezogen auf die in der Gärflüssigkeit enthaltene Menge an Oxalsäure, zugesetzt.

Als Folge eines solchen Überschusses wird das Reaktionsgleichgewicht günstigerweise in Richtung der Calciumoxalatbildung verschoben.

Es hat sich herausgestellt, daß eine Menge an Gips, die etwa der mehr als doppelten stöchiometrischen Menge entspricht, besonders günstig ist, doch ist sogar ein größerer Überschuß ohne negativen Einfluß.

Ein weiterer Vorteil des Zusatzes von Calciumsulfat im Überschuß ist in der Tatsache zu sehen, daß der sonst schmierige und daher sehr schlecht filtrierbare Calciumoxalat-Niederschlag durch die Anwesenheit von ungelöstem Calciumsulfat leicht filtrierbar wird, was gleichfalls die Effektivität der Oxalsäureentfernung erhöht.

Können in der Gärflüssigkeit vor Abtrennung des gefällten Calciumoxalates Calciumsulfatkristalle festgestellt werden, ist überdies auf einfachste Weise der Nachweis erbracht, daß eine ausreichende Menge an $CaSO_4$ zugegeben wurde.

Calciumsulfat kann beispielsweise in Form von Gips ($CaSO_4 . 2H_2O$) aber auch als Halbhydrat sowie als Anhydrit eingesetzt werden.

Die bei der Umsetzung mit Calciumsulfat in den Gärflüssigkeiten freigesetzte Schwefelsäure reagiert bei der anschließenden Fällung der Citronensäure mittels Kalkmilch ($Ca(OH)_2$), $CaO$ oder Calciumcarbonat mit diesen Fällungsmitteln und es entstehen daher anfänglich Gipskristalle in der Gärflüssigkeit, erst später fällt Calciumcitrat aus. Insbesondere bei den Fällungen von Calciumcitrat aus Gärflüssigkeiten auf Melassebasis war zu beobachten, daß die zunächst gebildeten Gipskristalle im Laufe der Fällung immer weniger wurden und zu Fällungsende völlig verschwunden waren.

Dies war umso überraschender, als bekannt ist, daß die Löslichkeit von $CaSO_4 . 2 H_2O$ mit der Temperatur und mit der Konzentration an Citronensäure in der Lösung steigt (N.I. Savchenko, Novotel'nova, N.Ya., Goma, I., G. und Petrova, L.V.; Khlebopek. Konditer. Prom. 17(7), 19 (1973)). Das Phänomen konnte somit nicht mit der verschiedenen Löslichkeit von Gips in den einzelnen Stadien der Fällung erklärt werden, da am Ende der Fällung infolge der geringeren Citronensäurekonzentration in der Lösung die Löslichkeit von Gips eher schlechter sein sollte.

Zur Klärung dieser Frage wurde folgender Versuch durchgeführt: 500 ml 10 %ige Citronensäurelösung wurden mit 3 ml konzentrierter Schwefelsäure versetzt und in der Hitze mit Kalkmilch gefällt. Anfänglich entstanden Gipskristalle, später dann Calciumcitratkristalle. Im Gegensatz zu den Fällungen aus Gärflüssigkeiten waren bei diesem Versuch die Gipskristalle bis zum Ende der Fällung in etwa gleicher Menge vorhanden.

Das Verschwinden der Gipskristalle bei den Fällungen aus Gärflüssigkeiten mußte somit anderen darin enthaltenen gelösten Komponenten zuzuschreiben sein.

Es stellte sich die Frage, welche Substanzen in den Gärflüssigkeiten imstande sind, mit relativ schwer löslichem Gips in Reaktion zu treten.

Nach eingehenden theoretischen Überlegungen, unter Berücksichtigung der Verhältnisse in industriellen Citronensäuremaischen, mußte angenommen werden, daß nur lösliche Salze der Citronensäure selbst, z.B. Alkalicitrate, mit dem Gips reagieren können.

Folgende Darlegungen sollen diesen Sachverhalt erhellen:

Zur Citronensäuregärung eingesetzte, übliche Rübenmelasse enthält beispielsweise durchschnittlich 10 bis 2 Massen% Asche, wovon ca. die Hälfte als $K_2O$ vorliegt und ca. 10 Massen% als $Na_2O$ (F. Reiff, Die Hefen, Band II, Seite 53, Verlag Hans Carl Nürnberg, 1962; H. Schiweck und L. Haberl, Einfluß der Zusammensetzung der Rübenmelasse auf die Hefeausbeute, "Zucker" 1973 (7), 347). Wenn man einen durchschnittlichen $K_2O$-Gehalt von 5,5 Massen% in der Melasse annimmt, so entspricht dies ca 4,5 Massen% Kalium.

Die Melasse muß, um submers vergoren werden zu können, im allgemeinen etwa im Verhältnis 1 : 4 verdünnt werden, so daß man annehmen kann, daß in der Gärflüssigkeit eine Konzentration von ca. 9 g Kalium/l vorliegt.

Es ist bekannt, daß das Kalium in der Melasse hauptsächlich an die organischen Säuren derselben gebunden vorliegt (F. Reiff, Die Hefen, Band II, Seite 53, Verlag Hans Carl Nürnberg, 1962; H. Olbrich, Die Melasse, Seite 22, Berlin 1956). Infolge der Aufnahme dieser Säuren, insbesondere der Aminosäuren, durch

3

den Mikroorganismus, z.B. Aspergillus niger, während der Gärung wird formal KOH frei. Das Kaliumhydroxid reagiert aber in einer citronensäurehältigen Gärflüssigkeit, sofern keine noch stärkeren Säuren in der Lösung anwesend sind, mit der Citronensäure. Es liegt somit in der Gärflüssigkeit formal Kaliumcitrat vor. Dieselben Überlegungen gelten sinngemäß auch für Natrium, Magnesium und Calcium.

Alkalicitrate sind aber durch beispielsweise Kalkmilch nicht in Calciumcitrat überführbar, sie bleiben am Ende einer Fällung der Citronensäure mit Kalkmilch in Lösung. Die Alkalicitrate sind nun die Reaktionspartner für den zu Beginn der Fällung aus Schwefelsäure bzw. gelösten Sulfaten entstandenen Gips. Nach der Gleichung

$$2\ K_3Cit\ +\ 3\ CaSO_4 \rightleftharpoons Ca_3Cit_2\ +\ 3\ K_2SO_4,$$

worin Cit für den Citronensäurerest steht, entsteht nun aus dem Gips Tricalciumcitrat. Die Löslichkeit von $CaSO_4.2H_2O$ beträgt in reinem Wasser bei 90° C 2,2 g/l und ist somit rund viermal so hoch wie jene von $Ca_3Cit_2.4H_2O$ unter denselben Bedingungen.

Aus den voranstehenden Darlegungen ist somit ersichtlich, daß die erfindungsgemäße Fällung der Oxalsäure durch Zusatz von Calciumsulfat nicht nur für die Entfernung der Oxalsäure selbst Vorteile bringt, sondern sich auch auf die anschließende Gewinnung der Citronensäure durch Fällung positiv auswirkt. Der in der von Oxalsäure befreiten Gärflüssigkeit zwangsläufig anfallende Gips ist in der Lage, im Vorliegen von löslichen Salzen der Citronensäure begründete Verluste an Citronensäure abzufangen.

Nach einer weiteren bevorzugten Ausführungsform des erfindungsgemäßen Verfahrens wird feinteiliges Calciumsulfat, welches bei der Gewinnung von Citronensäure aus den Gärflüssigkeiten durch Ausfällen von Tricalciumcitrat und Behandlung des Tricalciumcitrates mit Schwefelsäure als Nebenprodukt anfällt, eingesetzt.

Das bei dieser Behandlung in Form von Gips anfallende Calciumsulfat ist ohne weitere Vorbehandlung einsetzbar; es ist besonders oberflächenreich und reagiert daher sehr schnell mit der Oxalsäure. Zudem ist der Einsatz eines solchen - im Betrieb anders kaum wiederverwertbaren-Gipses auch aus Gründen des Umweltschutzes und der Prozeßökonomie (Verminderung des Chemikalienbedarfes) besonders vorteilhaft.

Die Erfindung wird durch die folgenden Modellversuche und Beispiele noch näher erläutert:

Modellversuch 1:

500 ml einer wässerigen Lösung mit einem Gehalt an 100 g Citronensäure/l sowie 20 g Oxalsäure . $2H_2O$/l und mit einem pH-Wert von 1,15 werden auf 80° C erhitzt, mit der zweifachen stöchiometrischen Menge an Gips, d.i. 27,4 g $CaSO_4$ . $2H_2O$ versetzt und durchgemischt. In Abständen von 5 min werden Proben gezogen, filtriert und in den Filtraten wird die freie Oxalsäure titrimetrisch bestimmt. Wie die nachstehende Tabelle zeigt, ist die Reaktion bei der angegebenen Temperatur bereits nach 5 min beendet.

| Zeit [min] | mg Oxalsäure . $2H_2O$/ml | % der eingesetzten Oxalsäure |
|---|---|---|
| 5 | 1,04 | 5,2 |
| 10 | 0,96 | 4,8 |
| 15 | 1,00 | 5,0 |
| 20 | 0,96 | 4,8 |
| 25 | 0,98 | 4,9 |

Ebenso ist ersichtlich, daß unter den gegebenen Bedingungen über 95 % der eingesetzten Oxalsäure entfernt werden konnten. Der pH-Wert des Versuchsansatzes sank während der Reaktion von 1,15 auf 0,8.

Modellversuch 2:

Die Menge der eingesetzten Oxalsäure wurde auf das Doppelte erhöht: 500 ml einer wässerigen

4

Lösung von 100 g Citronensäure/l und 40 g Oxalsäure . $2H_2O/l$ (pH-Wert = 1,1) wurden wie im Modellversuch 1 mit der entsprechenden (doppelten) Menge Gips, d.i. 54,6 g $CaSO_4$ . $2H_2O$, behandelt. Auch in diesem Fall wurden in der Lösung nach 5 min 0,97 mg Oxalsäure/ml entsprechend 2,4 % der eingesetzten Oxalsäure gefunden. Der pH-Wert betrug bei Versuchsende 0,8.

Beispiel:

500 ml einer Gärflüssigkeit, die nach Abfiltrieren des Mycels des Mikroorganismus (Aspergillus niger) einen Gehalt von 108 g Citronensäure/l und 16 g Oxalsäure . $2H_2O/l$ aufwies, wurden bei 85°C unter ständigem Rühren mit 30,6 g $CaSO_4$ . $2H_2O$ versetzt. In den analog Modellversuch 1 gezogenen Proben wurden nachstehende Werte ermittelt:

| Zeit [min] | mg Oxalsäure . $2H_2O/ml$ | % der eingesetzten Oxalsäure |
|---|---|---|
| 5 | 0,784 | 4,9 |
| 10 | 0,784 | 4,9 |
| 15 | 0,800 | 5,0 |
| 20 | 0,768 | 4,8 |
| 25 | 0,784 | 4,9 |

Auch in diesem Fall war die Reaktion somit nach 5 min beendet, die Menge an verbliebener Oxalsäure war nicht höher als in reinen Modellösungen. Der pH-Wert der Lösung sank von 2,1 vor der Fällung auf 1,9. Die feststoffhaltige Gärflüssigkeit erwies sich als rasch und verlustfrei filtrierbar.

Das Filtrat wurde auf 90°C erhitzt und Tricalciumcitrat durch Zugabe von Kalkmilch bis zu einem pH-Wert von 5,5 ausgefällt; das Tricalciumcitrat wurde durch Filtration auf einer Nutsche gesammelt und mit 300 ml Heißwasser gewaschen. In den vereinigten Filtraten wurde die noch vorhandene Citronensäuremenge analytisch erfaßt. Sie betrug 1,25 g, d.s. 2,3 % der in der vom Mycel befreiten Gärflüssigkeit enthaltenen Menge.

Vergleichsbeispiel:

500 ml der gleichen Gärflüssigkeit wie im vorstehenden Beispiel wurden mit Kalkmilch bis zum Erreichen eines pH-Wertes von 3,0 versetzt. Nach Abtrennen des entstandenen, schwer filtrierbaren Calciumoxalates wurde mit dem Filtrat genauso wie im obigen Beispiel weiter verfahren. Die Analyse ergab in den vereinigten Filtraten der Citratfällung einen Restgehalt von 2,34 g Citronensäure, d.s. 4,3 % der in der Gärflüssigkeit ursprünglich enthaltenen Menge.

Es ist ersichtlich, daß bei der Gewinnung von Citronensäure aus Gärflüssigkeiten, welche nach dem erfindungsgemäßen Verfahren von Oxalsäure befreit wurden, beträchtlich geringere Citronensäureverluste resultieren.

## Patentansprüche

1. Verfahren zur Entfernung von Oxalsäure aus sauren Gärflüssigkeiten von citronensäurebildenden Mikroorganismen durch Fällung der Oxalsäure als schwerlösliches Calciumoxalat und Abtrennung des Calciumoxalates, dadurch gekennzeichnet, daß die Fällung durch Zusatz von Calciumsulfat zu der Gärflüssigkeit vor oder nach Abscheidung der Mikroorganismenzellen vorgenommen wird.

2. Verfahren nach Anspruch 1, dadurch gekennzeichnet, daß die Fällung bei Temperaturen über 40°C, vorzugsweise zwischen 80 und 90°C, und mit einer Verweilzeit von mindestens 5 min durchgeführt wird.

**3.** Verfahren nach einem der Ansprüche 1 und 2, dadurch gekennzeichnet, daß Calciumsulfat im stöchiometrischen Überschuß, bezogen auf die in der Gärflüssigkeit enthaltene Menge an Oxalsäure, zugesetzt wird.

**4.** Verfahren nach einem der Ansprüche 1 bis 3, dadurch gekennzeichnet, daß feinteiliges Calciumsulfat, welches bei der Gewinnung von Citronensäure aus den Gärflüssigkeiten durch Ausfällen von Tricalciumcitrat und Behandlung des Tricalciumcitrates mit Schwefelsäure als Nebenprodukt anfällt, eingesetzt wird.

**Claims**

**1.** Method of removing oxalic acid from acidic fermentation liquids of citric-acid forming microorganisms by precipitation of the oxalic acid as a hardly soluble calcium oxalate and separation of the calcium oxalate, characterised in that the precipitation is carried out by the addition of calcium sulfate to the fermentation liquid prior to or after separation of the microorganism cells.

**2.** Method according to claim 1, characterised in that the precipitation is carried out at temperatures of above 40 °C, preferably between 80 and 90 °C, and with a residence time of at least 5 minutes.

**3.** Method according to one of claims 1 and 2, characterised in that calcium sulfate is added in stoichiometric excess, based on the amount of oxalic acid contained in the fermentation liquid.

**4.** Method according to one of claims 1 to 3, characterised in that fine particle calcium sulfate is used, which incurs as a by-product in the recovery of citric acid from the fermentation liquids by precipitating tricalcium citrate and treating the tricalcium citrate with sulfuric acid.

**Revendications**

**1.** Procédé pour éliminer l'acide oxalique contenu dans des liqudies de fermentation acides de micro-organismes formateurs d'acide citrique par précipitation de l'acide oxalique sous forme d'oxalate de calcium peu soluble et séparation de l'oxalate de calcium, caractérisé en ce que la précipitation est effectuée par addition de sulfate de calcium au liquide de fermentation avant ou après séparation des cellules des micro-organismes.

**2.** Procédé selon la revendication 1, caractérisé en ce que la précipitation est réalisée à des températures supérieures à 40 °C, de préférence entre 80 et 90 °C, et avec une durée d'au moins 5 min.

**3.** Procédé selon l'une des revendications 1 et 2, caractérisé en ce que le sulfate de calcium est ajouté en excès stoechiométrique par rapport à la quantité d'acide oxalique contenue dans le liquide de fermentation.

**4.** Procédé selon l'une des revendications 1 à 3, caractérisé en ce que l'on utilise du sulfate de calcium finement divisé qui apparaît sous forme de sous-produit lors de l'obtention de l'acide citrique à partir des liqudies de fermentation par précipitation du citrate tricalcique et traitement du citrate tricalcique par l'acide sulfurique.